## Europäisches Patentamt

## European Patent Office

## Office européen des brevets

(19)

(11) Veröffentlichungsnummer: **0 169 364**
**B1**

(12) # EUROPÄISCHE PATENTSCHRIFT

(45) Veröffentlichungstag der Patentschrift:
**03.08.88**

(21) Anmeldenummer: **85107404.7**

(22) Anmeldetag: **15.06.85**

(51) Int. Cl.⁴: **A 61 L 15/06** // A61K31/505

(54) **Selbstklebendes Pflaster.**

(30) Priorität: **23.06.84 DE 3423293**
**23.06.84 DE 3423328**

(43) Veröffentlichungstag der Anmeldung:
**29.01.86 Patentblatt 86/5**

(45) Bekanntmachung des Hinweises auf die Patenterteilung:
**03.08.88 Patentblatt 88/31**

(84) Benannte Vertragsstaaten:
**AT BE CH DE FR GB IT LI NL SE**

(56) Entgegenhaltungen:
**EP - A - 0 020 905**

**Die Akte enthält technische Angaben, die nach dem
Eingang der Anmeldung eingereicht wurden und die
nicht in dieser Patentschrift enthalten sind.**

(73) Patentinhaber: **Beiersdorf Aktiengesellschaft,
Unnastrasse 48, D-2000 Hamburg 20 (DE)**

(72) Erfinder: **Guse, Günter, Dr., Liliencronstrasse 30,
D-2000 Hamburg 73 (DE)**
Erfinder: **Stenzel, Wolfgang, Dr., Lerchenweg 8,
D-2057 Reinbek (DE)**
Erfinder: **Asmussen, Bodo, Dr., Dorotheenweg 1a,
D-2071 Ammersbek (DE)**

## Beschreibung

Die Erfindung betrifft ein selbstklebendes Pflaster mit einem Wirkstoff zur transdermalen Applikation, das im Folgenden näher definiert ist.

Derartige selbstklebende Pflaster zur transdermalen Applikation von Wirkstoffen sind bekannt. Unter den Wirkstoffen befindet sich auch das Antihypertonikum Clonidin. Bei diesen bekannten Pflastern kann man zwei Typen von Pflastern unterscheiden. Der eine bekannte Pflastertyp ist gekennzeichnet durch ein Wirkstoffreservoir und eine Steuermembran, durch welche die Abgabe des Wirkstoffs in die Haut dosiert wird. Der zweite Typ der bekannten Pflaster enthält den Wirkstoff in einer mehr oder weniger klebenden Matrix, die direkt auf die Haut aufzubringen ist.

Die bekannten, Antihypertonika enthaltenden Pflaster, weisen nicht immer voll zufriedenstellende Eigenschaften auf. So wird beispielsweise der Nutzen dieser Therapieform durch Nebenwirkungen oder beispielsweise allergische Hautreaktionen eingeschränkt.

Aufgabe der Erfindung war es, hier Abhilfe zu schaffen und ein Pflaster, das diese Nachteile des Standes der Technik nicht aufweist, zu schaffen. Insbesondere war ein Pflaster zu schaffen, das keine oder nur geringe Nebenwirkungen und Hautreaktionen bewirkt und das daher auch besonders vorteilhaft für längere Applikationszeiten, wie zum Beispiel eine Woche, geeignet ist und eine starke, konstante antihypertensive Wirkung aufweist.

Diese Aufgabe wird gelöst durch ein mit einer Selbstklebemasse versehenes, wirkstoffhaltiges Pflaster für die transdermale Applikation des Wirkstoffes, das dadurch gekennzeichnet ist, dass der Wirkstoff Moxonidin ist.

Der Wirkstoff Moxonidin (2-Methyl-4-chlor-6-methoxy-5- (2-imidazolin-2-yl)- amino-pyrimidin) ist in der US-A-4 323 570 als Antihypertonikum beschrieben. Er erfüllt in unerwarteter Weise alle Anforderungen, die an einen für die antihypertensive Therapie bestimmten Wirkstoff bei transdermaler Applikation gestellt werden. Insbesondere besitzt er die Fähigkeit, mit ausreichender Geschwindigkeit in therapeutisch wirksamer Menge relativ kleine Hautflächen zu durchdringen, ohne – selbst bei längerem Hautkontakt – Irritation und Sensibilisierung zu verursachen.

Das erfindungsgemässe Pflaster ist zur transdermalen Applikation von Moxonidin bestimmt. Als besonders vorteilhaft hat sich die einmal wöchentliche Applikation des erfindungsgemässen Pflasters erwiesen. Es sind jedoch auch kürzere Intervalle möglich, etwa einmal bis zweimal täglich.

Als günstig für die Hypertoniebehandlung beim Menschen mit dem erfindungsgemässen Pflaster hat sich eine täglich freigesetzte Wirkstoffmenge von 0,1 bis 10 mg Moxonidin erwiesen, besonders günstig 0,5 bis 5 mg über 24 Stunden. Ganz besonders bevorzugt werden 1,5 bis 2,5 mg/24 Stunden.

Geeignete transdermale therapeutische Systeme in der Form eines Pflasters können nach unterschiedlichen Bauprinzipien hergestellt werden. In der Regel vereinigen sie die Funktionen Wirkstoffreservoir, Freisetzungssteuerung und Selbsthaftung auf der Haut. Je nach Anordnung dieser Elemente wird zwischen Matrixsystemen und sogenannten Membranmodellen unterschieden. Bei letzteren liegen Wirkstoffreservoir, Steuermembran und die Selbstklebemasse getrennt vor, während Matrixsysteme, insbesondere bei einschichtigem Aufbau, diese Trennung nicht zeigen. Auch Kombinationen dieser Pflastertypen werden bevorzugt. Es ist auch möglich, zu einer ganz anderen Anordnung dieser Elemente dadurch zu kommen, dass man wirkstofftragende und selbstklebende Bereiche getrennt anordnet. Dies ist beispielsweise durch Druckverfahren, insbesondere den Siebdruck möglich. Eine weitere Form der getrennten Anordnung ist dadurch gegeben, dass man den Wirkstoff in unterschiedliche dermatologisch gebräuchliche Vehikel einarbeitet, diese auf Trägerstoffe wie Vlies- oder Mullkissen bringt und sie mit geeigneten selbstklebenden Deckpflastern an der Applikationsstelle fixiert.

Die Auswahl der Selbstklebemassen für erfindungsgemässe Pflaster ist nicht eingeschränkt. Jede Selbstklebemasse ist geeignet, in die der Wirkstoff sich einarbeiten lässt, die die Stabilität des Wirkstoffs auch während längerer Lagerungszeiten nicht beeinträchtigt, die während der Anwendung sicher auf der Haut haftet und dabei den Wirkstoff in gewünschter Weise freisetzt oder die für den Wirkstoff eine ausreichende Durchlässigkeit aufweist.

Besonders geeignete transdermale therapeutische Systeme in der Form eines Pflasters sind die des Matrix-Typs.

Auch die Auswahl der Selbstklebemassen für erfindungsgemässe Pflaster vom Matrix-Typ ist nicht eingeschränkt. Jede Selbstklebemasse ist geeignet, in die der Wirkstoff sich einarbeiten lässt, die die Stabilität des Wirkstoffs auch während längerer Lagerungszeiten nicht beeinträchtigt, die während der Anwendung sicher auf der Haut haftet und dabei den Wirkstoff in gewünschter Weise freisetzt.

Mit besonderem Vorteil werden Polyacrylat-Selbstklebemassen oder solche auf der Basis von gesättigten Kautschuken wie Polyisobuten und Klebharzen verwendet. Geeignet sind beispielsweise Haftkleber auf der Basis von Acryl- und Methacrylsäureestern mit Alkylresten von 4 bis 18 C-Atomen wie Butyl-, 2-Ethylhexyl- oder Stearylacrylat, gewünschtenfalls mit einem geringeren Anteil von 0,1–10 Gewichtsprozent eines polaren Comonomeren wie Acrylsäure. Unter den Haftklebern auf Basis Polyisobuten/Klebharz sind solche besonders gut geeignet, die einen hochmolekularen gerüstbildenden neben einem niedermolekularen klebrigmachenden Polyisobuten-Anteil enthalten.

Besonders bevorzugt sind Polyisobutenmassen, die eine höhermolekulare, gerüstbildende Komponente mit einem mittleren Molekulargewicht von 2 400 000–3 200 000, insbesondere 2 800 000 von 85–40 Gew.-%, insbesondere von 55–45 Gew.-% und eine niedermolekulare, klebrigmachende, weichmachende Komponente mit einem mittleren Molekulargewicht von 30 000–50 000, insbesondere 40 000 von 15–60 Gew.-%, insbesondere 45–55 Gew.-% enthalten. Das klebrigmachende Harz ist vorzugsweise ein aliphatisches Kohlenwasserstoffharz.

Weitere bevorzugte Selbstklebemassen sind Polyurethan-, Polysiloxan- und Polyvinylalkoholmassen, insbesondere Polyvinylalkohol-Gele.

Den Selbstklebemassen, insbesondere Polyacrylatklebemassen und gesättigten Kautschuken, können Zusätze, vorzugsweise in Anteilen bis zur fünffachen Wirkstoffmenge (Gewichtsmenge), beigegeben werden, die die Klebeigenschaften und/oder die Wirkstoffliberation in gewünschter Weise beeinflussen. Dies können je nach liberationskinetischem Erfordernis und gewünschter Anwendungsdauer des Pflasters polare bzw. hydrophile oder weniger polare und unpolare Zuschlagstoffe sein.

Besonders vorteilhaft für die Freisetzung von Moxonidin aus der Polymermatrix, sind polare bzw. hydrophile Substanzen, vornehmlich mehrwertige Alkohole wie beispielsweise Glycerin, Propandiol-1,2 oder Polyethylenglykole, insbesondere solche mit einem Molekulargewicht von 200 bis 800.

Als weniger polare oder unpolare Hilfsstoffe kommen vorzugsweise Triglyceride mittelkettiger Fettsäuren, Fettsäureester einwertiger Alkohole oder Paraffinöle in Betracht.

Die Herstellung der Selbstklebemassen für die erfindungsgemässen Pflaster kann nach an sich bekannten Techniken erfolgen. Polyacrylate werden vorteilhaft durch Lösungspolymerisation im Rührkessel hergestellt, Kautschuk-Harz-Gemische mit Lösungsmitteln in Kneter.

Der Wirkstoff Moxonidin kann der jeweiligen Polymerlösung in mikronisierter Form oder – mit besonderem Vorteil – in gelöstem Zustand beigefügt werden. Im letzteren Falle liegt der Wirkstoff nach dem Verdampfen des Lösungsmittels in äusserst feiner Verteilung vor, wodurch die Liberation signifikant erhöht wird.

Das Aufbringen der wirkstoffhaltigen Selbstklebemassen auf flächige Träger, insbesondere diffusionsdichte Folien, zur Erzeugung von Matrix-Pflaster kann nach an sich bekannten Techniken erfolgen. Vorzugsweise werden diese Massen mit Walzenauftragsverfahren, Streichbalken oder Rakeln, insbesondere Drahtrakeln, aufgebracht.

Aus der beschichteten und getrockneten Bahn können dann einzelne Pflaster ausgestanzt, mit einer wiederabziehbaren Schutzfolie eingedeckt und in einem Siegelbeutel verpackt werden.

Die Wirkstoffliberation und ihr zeitlicher Verlauf können auch über die Auflagefläche des Pflasters, die Schichtdicke der Matrix und deren Wirkstoffgehalt beeinflusst werden. Diese Grössen können vorteilhaft je nach therapeutischem Erfordernis aufeinander abgestimmt werden. Die Auflagefläche wird ausserdem auch von der Notwendigkeit der bequemen und sicheren Handhabung und der faltenfreien Applikation mitbestimmt. Sie soll nicht unter 1 cm² und nicht über 50 cm² liegen, vorzugsweise im Bereich von 3 bis 20 cm² liegen, vorzugsweise im Bereich von 3 bis 20 cm². Die Schichtdicke wird so bemessen, dass einerseits ein ausreichendes Mindestreservoir an Wirkstoff je Flächeneinheit vorliegt, andererseits aber die Trocknung der Schicht nicht unnötig verzögert wird. Der praktisch anwendbare Bereich reicht etwa von 20 bis 500 g/m², bevorzugt von 50 bis 200 g/m². Die Konzentration des Wirkstoffes, bezogen auf das Gesamtgewicht der Matrix, soll 2 bis 20 Gew.-%, vorzugsweise 5 bis 15 Gew.-%, betragen.

Wünscht man einen Kontakt zwischen Wirkstoff und Selbstklebemasse gänzlich zu vermeiden, so ist es zur Ausschaltung jeder nur denkbaren Wechselwirkung auch möglich, auf einem flächigen Träger diskrete Wirkstoff-Segmente und separate Klebstoff-Segmente anzuordnen. Dabei werden sowohl die Wirkstoff- als auch die Klebstoff-Segmente jeweils aus Dispersionen mit hohem Feststoffgehalt im Druckverfahren aufgetragen. Als Druckverfahren eignen sich dabei der Tiefdruck, insbesondere aber der Siebdruck, der sich durch einfache Handhabung und ausserordentliche Wirtschaftlichkeit bei exakter Anordnung der Segmente auszeichnet.

Demgemäss betrifft diese Ausgestaltung der Erfindung ein selbstklebendes Pflaster mit separaten Wirkstoff-Segmenten auf einem Träger zur transdermalen Applikation, das dadurch gekennzeichnet ist, das auf dem Träger räumlich von den Wirkstoff-Segmenten separate Klebstoff-Segmente angeordnet sind, die aus solchen Klebstoff-Systemen bestehen, die sich als Dispersion, Plastisol oder Organosol verarbeiten lassen, wobei sowohl die Klebstoff-Segmente als auch die Wirkstoff-Segmente angenähert kalottenförmig sind und mit ihrer Basis auf dem Träger haften.

Besonders vorteilhaft ist es, dass die Scheitelpunkte der Wirkstoff- und Klebstoff-Segmente gegenüber dem Träger etwa gleiche Höhe haben. Damit wird ein gleichmässig guter Kontakt zur Hautoberfläche beider Elemente erreicht.

Bevorzugt sind die Klebstoff-Segmente und die Wirkstoff-Segmente in regelmässiger Folge angeordnet, bedingt durch die Geometrie der Siebdruck-Vorrichtungen. Dabei haben die Kalotten vorzugsweise einen Basisdurchmesser von bis zu 5000 µm, insbesondere 200–1500 µm. Falls erwünscht, lassen sich jedoch durchaus auch grössere Kalotten herstellen.

Auch für diese Ausgestaltung der Erfindung sind zahlreiche an sich bekannte Selbstklebemassen geeignet, unter anderem Kautschuk, Polyacrylsäureester oder Polyisobutylen, gegebenenfalls zusammen mit klebrigmachenden Harzen. Diese Kleber werden vorteilhaft aus wässrigen, konzentrierten, thixotropen Haftklebe-Dispersionen verarbeitet, wobei der Feststoffgehalt vorzugsweise bei 55–65 Gew.-% liegt. Auch hier sind beispielsweise Haftkleber auf der Basis von (Meth)-Acrylsäureestern mit Alkylresten von 4–18 C-Atomen geeignet, wie Acrylsäurebutylester, Acrylsäure-2-ethylhexylester oder Acrylsäure-stearylester, vernetzt oder unvernetzt, wobei eine Vernetzung gegebenenfalls durch Elektronenstrahlen bewirkt sein kann.

Das Prinzip des Rotationssiebdrucks besteht in der Verwendung einer rotierenden, nahtlosen, trommelförmigen und perforierten Rundschablone. Im Innenmantel presst eine mechanisch oder magnetisch gehaltene Rund- oder Vierkantrakel die in die Trommel eingespeiste Dispersion durch die Perforation der Schablonenwand auf die Trägerbahn. Diese wird mit einer Geschwindigkeit geführt, die der Umfangsgeschwindigkeit der rotierenden Siebtrommel entspricht, mittels einer Gegendruckwalze gegen den Aussenmantel der Siebtrommel. Zur Herstellung der erfindungsgemässen Pflaster werden dabei in geeigneter Weise zwei hintereinander geschaltete, synchron laufende Siebdruckwerke verwendet, die Klebstoff- und dann Wirkstoff-Zubereitungen entsprechend der Lochgeometrie der Siebe passgenau nebeneinander plazieren. Grösse und Dosierung der sich als Kalotten ausbildenden mikrokleinen Kleb- und Wirkstoff-Flächen lässt sich durch den Durchmesser des Siebloches und der Wandstärke des Siebes genau bestimmen. Die Zahl der Möglichkeiten der Anordnung ist unbegrenzt und kann je nach Wunsch gewählt werden. Damit ergibt sich zudem als wirtschaftlicher Vorteil der Herstellungsweise, dass Klebstoff- und Wirkstoffzubereitung in einer Linie aufgebracht werden können, und dies mit hoher Dosierungsgenauigkeit.

Die Dispersionen sollten gelartig bis pastös sein und nach Auftrocknung einen elastischen Film bilden, z.B. durch Zusatz von Polyvinylalkohol, Polyvinylpyrrolidon, wasserlöslichen Cellulosederivaten oder anderen mehr oder weniger wasserlöslichen Verfilmungsmitteln.

Soll eine Elektronenstrahl-Vernetzung erfolgen, so geschieht dies vorteilhaft nach dem Klebstoffauftrag und vor dem Wirkstoffauftrag, ohne die Produktionslinie zu unterbrechen.

Abschliessend wird die Beschichtung vorteilhaft in einem Heissluftkanal oder durch Infrarot- bzw. Hochfrequenzstrahlung getrocknet. Als Träger kommen auch in diesem Falle insbesondere diffusionsdichte Folien in Betracht. Geeignet sind insbesondere Träger, auf denen Klebstoff und Wirkstoffzubereitung ohne Hilfsmittel haften. Gegebenenfalls kann die Oberfläche des Trägers aber auch zusätzlich behandelt werden, z.B. durch Korona-Entladung oder Beschichtung mit einem Haftvermittler, der als Primer die Verankerung vermittelt.

Eine weitere Möglichkeit, Wechselwirkungen zwischen der Wirkstoffzubereitung und der Selbstklebemasse auszuschliessen, besteht erfindungsgemäss darin, zwischen einer im wesentlichen geschlossenen Kleberschicht und diskreten Wirkstoff-Segmenten einen Trennfilm anzuordnen.

Demgemäss betrifft eine weitere Ausbildung der Erfindung ein selbstklebendes Pflaster mit separaten Wirkstoff-Segmenten auf einem klebstoffbeschichteten Träger zur transdermalen Applikation, das dadurch gekennzeichnet ist, dass sich zwischen den Wirkstoff-Segmenten und der Klebstoffschicht ein Trennfilm befindet.

Dieser Trennfilm besteht vorzugsweise aus einer Membran, die störende Wechselwirkungen zwischen Wirkstoffzubereitung und Kleberschicht ausschliesst, also im Sinne der Funktion des erfindungsgemässen Gegenstandes nichtpermeabel ist. In besonders geeigneter Weise ist dieser Trennfilm z.B. aus Polyvinylidenchlorid oder Copolymeren davon und auch das auf ihm angeordnete Wirkstoff-Segment aus jeweils einer Dispersion mit hohem Feststoffgehalt im Druckverfahren vom Typ Tiefdruck oder insbesondere Siebdruck aufgetragen.

Damit wird erfindungsgemäss erreicht, dass es zu einer Interaktion zwischen Wirkstoff und Kleber nicht kommen kann.

Die Zusammensetzung des Klebers braucht auch bei dieser Ausgestaltung der Erfindung keine Rücksicht mehr auf den Wirkstoff zu nehmen. Es können übliche Selbstklebemassen verwendet werden, insbesondere hautfreundliche derartige Massen, wie solche auf Acrylat-Basis. Zur Verankerung dieser Kleber auf dem Träger können gegebenenfalls Haftvermittler verwendet werden, die als Vorstrich aufgetragen werden.

Sollen als Klebemassen elektronenstrahlvernetzte Massen verwendet werden, so wird die Vernetzung vorzugsweise durchgeführt, bevor die Wirkstoffzubereitung auf die Segmente des Trennfilms aufgebracht wird. Dies kann vorteilhaft in Linie erfolgen, wobei zunächst in an sich bekannter Weise der Klebstoff auf den Träger beschichtet wird, dann im Siebdruckverfahren die Segmente des Trennfilms aufgebracht werden, worauf vernetzt wird, um schliesslich auf die Segmente des Trennfilms im Siebdruck die Wirkstoffzubereitung aufzutragen. Vorteilhaft ist es, wenn die Trennfilm-Segmente einen etwas grösseren Durchmesser als die Wirkstoffpunkte haben.

Die Wirkstoffzubereitung enthält filmbildende und andere geeignete pharmazeutische Hilfsstoffe, die dem Fixieren auf dem Trennfilm und der optimalen Wirkstoffdiffusion in die Haut dienlich sind.

Vorteilhaft sind die Wirkstoff-Segmente in regelmässiger Folge angeordnet und haben einen Durchmesser bis zu 5000 μm, insbesondere 200–1500 μm.

Im folgenden wird die Erfindung beispielhaft erläutert, ohne damit aber ihren Gegenstand unnötig einschränken zu wollen (GT = Gewichtsteile, Prozentangaben sind Gewichtsprozente):

Beispiel 1

A. Herstellung einer Wirkstofflösung

1,0 GT Moxonidin und 0,6 GT Polyethylenglycol 400 werden in einem Gemisch von 65 GT

1,1,1-Trichlorethan und 35 GT Ethanol bei 40 °C klar gelöst.

## B. Herstellung einer Klebstofflösung

Ein Copolymerisat aus 97,2 Gew.-% Butylacrylat und 2,8 Gew.-% Acrylsäure wird durch Lösungspolymerisation in Benzin/Aceton hergestellt (K-Wert 76). 27,4 GT dieser Lösung, deren Festkörpergehalt 38 Gew.-% beträgt, werden mit 22 GT Toluol verdünnt und auf 40 °C erwärmt.

## C. Beschichtung

Die beiden 40 °C warmen Lösungen werden vereinigt und unter ständigem Rühren langsam auf Raumtemperatur abgekühlt. Die so entstehende streichfertige klare Lösung hat einen Moxonidin-Gehalt von 0,66 Gew.-%. Mit Hilfe eines geeigneten Drahtrakels wird je eine Schicht der wirkstoffhaltigen Selbstklebemasse auf zwei Polyethylenterephthalat-Folien erzeugt (eine 15 μm starke Folie als Träger und eine siliconisierte 100 μm starke Folie als wiederentfernbare Schutzabdeckung). Beide werden 24 Stunden bei Raumtemperatur getrocknet und dann zusammenkaschiert, so dass insgesamt eine Trockenschichtdicke von 91,8 g/m² entsteht.

## D. Konfektionierung

Aus der getrockneten und mit der Siliconfolie bedeckten Bahn werden durch Schneiden oder Stanzen Pflaster beliebiger Grösse hergestellt, die einen Moxonidin-Gehalt von 0,765 mg/cm² besitzen. Sie werden einzeln in ein diffusionsdichtes Primärpackmittel eingeschweisst, beispielsweise einen Flachbeutel aus Polyethylen/Aluminium/Papier-Verbundmaterial.

## E. Liberation

Ein derartiges Pflaster mit einer Fläche von 16 cm², entsprechend 12,24 mg Moxonidin, liberiert in vitro bei 34 °C unter Standard-Bedingungen binnen 24 Stunden 5,15 mg Moxonidin in isotonische Kochsalzlösung.

## Beispiel 2

Es wird ein Pflaster wie in Beispiel 1 beschrieben hergestellt, mit der einzigen Variation, dass das gelöste Moxonidin durch die gleiche Menge mikronisiertes Moxonidin ersetzt ist. Liberation entsprechend Beispiel 1 bestimmt: 4,36 mg/24 h.

## Beispiel 3

Es wird ein Pflaster wie in Beispiel 1 beschrieben hergestellt, mit der einzigen Variation, dass der Zusatz von Polyethylenglycol 400 unterbleibt. Liberation entsprechend Beispiel 1 bestimmt: 4,32 mg/24 h.

## Beispiel 4

Es wird ein Pflaster wie in Beispiel 1 beschrieben hergestellt, mit der einzigen Variation, dass die Acrylat-Selbstklebemasse durch eine solche auf Basis von hochmolekularem Polyisobuten und aliphatischem Kohlenwasserstoffharz (Escorez® 1202, Esso AG), Erweichungspunkt 97 °C, (hergestellt im Kneter) ersetzt wird. Liberation entsprechend Beispiel 1 bestimmt: 1,37 mg/24 Stunden.

## Beispiel 4a

Es wird ein Pflaster wie im Beispiel 4 beschrieben hergestellt mit der Variation, dass die Klebemasse folgende Zusammensetzung hat:

24,9 GT Polyisobutylen $\overline{M}_v$ = 2 800 000 (im Handel erhältlich als Oppanol® B 150)

26,3 GT Polyisobutylen $\overline{M}_v$ = 40 000 (Oppanol® B 10)

22,6 GT aliphatisches Kohlenwasserstoffharz (Escorez® 1202, Esso AG), Erweichungspunkt R+K 97 °C

## Beispiel 5

A. Herstellung einer Wirkstoff-Zubereitung

27,35 Gewichtsteile Hydroxypropylmethylcellulose (Pharmacoat® 603) werden in ein Gemisch von 30 GT Isopropanol, 30 GT Wasser und 8,20 GT 1,2-Propylenglycol kalt eingerührt. Das Gemisch wird unter ständigem Rühren auf 50 °C erwärmt, bis eine klare Lösung entstanden ist. In diese werden 4,45 GT mikronisiertes Moxonidin sorgfältig eingemischt.

B. Herstellung einer Klebstoffzubereitung

100 GT einer handelsüblichen wässrigen Polymerdispersion auf Acrylsäureester-Basis (Acronal® 80 D) werden durch Zusatz von 4 GT der 16%igen ammoniakalisch-wässrigen Lösung einer Polyarylsäure (Collacral® P) auf eine Viscosität von etwa 200 Pas verdickt.

## C. Druckvorgang

Eine einseitig aluminisierte und hautfarben lakkierte Polyethylenterephthalat-Folie von 15 μm Stärke (Hostaphan® RN 15) wird durch ein erstes rotierendes Siebdruckwerk bei einer Bahngeschwindigkeit von 20 m/Min mit der Wirkstoffzubereitung beschichtet. Dabei wird ein Siebzylinder verwendet, der Punktreihen in Bahnlängsrichtung erzeugt. Jeder Punkt hat einen Basisdurchmesser von etwa 400 μm; der Abstand zwischen den Punkten beträgt in Bahnlängsrichtung etwa 100 μm, in Querrichtung etwa 600 μm. Die Auftragsmenge wird über die Rakeleinstellung so geregelt, dass kalottenförmige Punkte entstehen, die im getrockneten Zustand eine Höhe von etwa 160 μm besitzen. Das entspricht einer Auftragsmenge von etwa 25 g Wirkstoffzubereitung je m² Träger. Die Trocknung erfolgt in einem Warmluftkanal von 6 m Länge bei etwa 70 °C. Ein zweites rotierendes Siebdruckwerk gleichen Typs druckt anschliessend Punktreihen der Klebstoffzubereitung zwischen die getrockneten wirkstoffhaltigen Punktreihen. Dabei werden Punktgrösse, Punkthöhe und somit auch die Gesamtauftragsmenge auf die gleichen Werte wie bei der Wirkstoffzubereitung eingeregelt. Hinter diesem zweiten Druckwerk folgt erneut eine Trockenstrecke des beschriebenen Typs und anschliessend ein Kaschierwerk, in dem die Bahn mit einem wiederentfernbaren Schutzfilm abgedeckt wird, beispielsweise einer einseitig aluminisierten und siliconisierten Polyethylenterephthalat-Folie von 100 µm Stärke (Hostaphan® RN 100).

D. Konfektionierung

Die zweifach bedruckte, getrocknete und abgedeckte Bahn wird nun in Einzelpflaster beliebiger Grösse aufgeteilt, je nach der erwünschten Wirkstoffdosierung. Wählt man beispielsweise eine Grösse von 25 cm³, so hat man je etwa 5000 Wirkstoff- und Klebstoffpunkte, entsprechend einem Gehalt von 7 mg Moxonidin. Diese Pflaster werden einzeln in ein diffusionsdichtes Primärpackmittel eingeschweisst, beispielsweise einen Flachbeutel aus PE/Alu/Papier-Verbundmaterial.

Beispiel 6

A. Herstellung einer Wirkstoffzubereitung 27,35 GT Hydroxypropylmethylcellulose (Pharmacoat® 603) werden in ein Gemisch von 30 GT Isopropanol, 30 GT Wasser und 8,20 GT 1,2-Propylenglycol kalt eingerührt. Das Gemisch wird unter ständigem Rühren auf 50 °C erwärmt, bis eine klare Lösung entstanden ist. In diese werden 4,45 GT mikronisiertes Moxonidin sorgfältig eingemischt.

B. Herstellung einer Trennfilmzubereitung 100 GT einer handelsüblichen wässrigen Polyvinylidenchlorid-Dispersion (Diofan® 190 D) werden durch Zusatz von 2 GT einer 10%igen wässrigen Lösung des Ammoniumsalzes einer Polyacrylsäure (Latekoll® AS) auf eine Viskosität von annähernd 100 Pas verdickt.

C. Vorbereitung der Trägerbahn

Zunächst wird eine Trägerbahn aus einseitig aluminisierter und hautfarben lackierter Polyethylenterephthalat-Folie von 15 µm Stärke (Hostaphan® RN 15) mit einer Selbstklebemasse vollflächig beschichtet, so dass sich ein Trocken-Auftragsgewicht von etwa 35 g/m² ergibt. Gut geeignet ist beispielsweise eine Selbstklebemasse auf Acrylsäureester-Basis gemäss DE-C-2 743 979.

D. Druckvorgang

Auf die getrocknete und vernetzte Selbstklebeschicht wird mit einem ersten rotierenden Siebdruckwerk bei einer Bahngeschwindigkeit von 20 m/Min. die Trennfilmzubereitung aufgetragen. Dabei wird ein Siebzylinder verwendet, der je Quadratzentimeter 200 Punkte von je 500 µm Basisdurchmesser in regelmässiger Anordnung erzeugt. Die Auftragsmenge wird über die Rakeleinstellung so geregelt, dass flache, fest auf der Polyacrylatschicht haftende PVDC-Filmsegmente entstehen, die im getrockneten Zustand eine Dicke von etwa 20 µm besitzen. Die Trocknung erfolgt in einem Warmluft-Trockenkanal von 6 m Länge bei etwa 70 °C.

Ein zweites rotierendes Siebdruckwerk gleichen Typs druckt anschliessend auf die PVDC-Filmsegmente passergenau die Wirkstoffpunkte von je 400 µm Basisdurchmesser. Die Auftragsmenge wird über die Rakeleinstellung so geregelt, dass die kalottenförmigen Punkte im getrockneten Zustand eine Höhe von etwa 160 µm besitzen. Das entspricht einer Auftragsmenge von etwa 25 g Wirkstoffzubereitung je m² Träger. Hinter diesem zweiten Druckwerk folgt erneut eine Trockenstrecke des beschriebenen Typs und anschliessend ein Kaschierwerk.

E. Konfektionierung

Die zweifach bedruckte, getrocknete und abgedeckte Materialbahn kann nun im Einzelpflaster beliebiger Grösse aufgeteilt werden, je nach der erwünschten Wirkstoffdosierung. Wählt man beispielsweise eine Grösse von 25 cm², so hat man auf dieser Fläche etwa 5000 Wirkstoffpunkte entsprechend einem Gehalt von 7 mg Moxonidin [2-Methyl-4-chlor-6-methoxy-5-(2-imidazolin-2-yl)-amino-pyrimidin]. Diese Pflaster werden einzeln in ein diffusionsdichtes Primärpackmittel eingeschweisst, beispielsweise einen Flachbeutel aus PE/Alu/Papier-Verbundmaterial.

**Patentansprüche**

1. Mit einer Selbstklebemasse versehenes, wirkstoffhaltiges Pflaster für die transdermale Applikation des Wirkstoffes, dadurch gekennzeichnet, dass der Wirkstoff Moxonidin (2-Methyl-4-chlor-6-methoxy-5-(2-imidazolin-2-yl)-amino-pyrimidin) ist.

2. Pflaster gemäss Anspruch 1, dadurch gekennzeichnet, dass es ein Matrix-Pflaster ist.

3. Pflaster gemäss Anspruch 1, dadurch gekennzeichnet, dass die Selbstklebemasse aus der Gruppe der Polyacrylat-, Polyisobuten-, Polyurethan-, Polysiloxan- und Polyvinylalkoholmassen ausgewählt ist.

4. Pflaster gemäss Anspruch 1, gekennzeichnet durch Selbstklebemassen auf der Basis von Acrylsäure- und Methacrylsäureestern mit Alkylresten von 4 bis 18 C-Atomen.

5. Pflaster gemäss Anspruch 1, gekennzeichnet durch Selbstklebemassen auf der Basis von Acrylsäure- und Methacrylsäureestern mit einem Gehalt von 0,1 bis 10 Gewichtsprozent an polaren Comonomeren.

6. Pflaster gemäss Anspruch 1, gekennzeichnet durch Selbstklebemassen auf der Basis von Polyisobuten/Klebharz mit einem hochmolekularen gerüstbildenden Anteil neben einem niedermolekularen Polyisobutylen-Anteil.

7. Pflaster gemäss Anspruch 1, gekennzeichnet durch hydrophile bzw. polare Substanzen als Zusätze zu den Selbstklebemassen.

8. Pflaster gemäss Anspruch 1 zur Behandlung der Hypertonie.

9. Selbstklebendes Pflaster mit separaten Wirkstoff-Segmenten auf einem Träger zur transdermalen Applikation, nach Anspruch 1 dadurch gekennzeichnet, dass auf dem Träger räumlich von den Wirkstoff-Segmenten separate Klebstoff-Segmente angeordnet sind, die aus solchen Klebstoffsystemen bestehen, die sich als Dispersion, Plastisol oder Organosol verarbeiten lassen, und wobei sowohl die Klebstoff-Segmente als auch die Wirkstoff-Segmente angenähert kalottenförmig sind und mit ihrer Basis auf dem Träger haften.

10. Selbstklebendes Pflaster mit separaten Wirkstoff-Segmenten auf einem Klebstoff-beschichteten Träger zur transdermalen Applikation, nach Anspruch 9 dadurch gekennzeichnet, dass sich zwischen den Wirkstoff-Segmenten und der Klebstoffschicht ein Trennfilm befindet.

## Revendications

1. Pansement contenant un principe actif, pourvu d'une masse auto-adhésive, pour l'application transcutanée du principe actif, caractérisé en ce que le principe actif est la moxonidine (2-méthyl-4-chloro-6-méthoxy-5- (2-imidazoline-2-yl)-amino-pyrimidine).

2. Pansement selon la revendication 1, caractérisé en ce que c'est un pansement à matrice.

3. Pansement selon la revendication 1, caractérisé en ce que la masse auto-adhésive est choisie parmi le groupe constitué par les masses de polyacrylate, de polyisobutène, de polyuréthane, de polysiloxane, et d'alcool polyvinylique.

4. Pansement selon la revendication 1, caractérisé par des masses auto-adhésives à base d'esters d'acide acrylique et d'acide méthacrylique avec des restes alkyles ayant de 4 à 18 atomes de carbone.

5. Pansement selon la revendication 1, caractérisé par des masses auto-adhésives à base d'esters d'acide acrylique et d'acide méthacrylique avec une teneur en comonomères polaires de 0,1 à 10% en poids.

6. Pansement selon la revendication 1, caractérisé par des masses auto-adhésives à base de polyisobutène/résine adhésive avec un élément structural de poids moléculaire élevé, jointe à un élément de polyisobutylène de faible poids moléculaire.

7. Pansement selon la revendication 1, caractérisé par des substances hydrophiles ou polaires en tant qu'additifs pour les masses auto-adhésives.

8. Pansement selon la revendication 1 pour le traitement de l'hypertension.

9. Pansement auto-adhésif muni de segments de principe actif séparés sur un support en vue de l'application transcutanée, selon la revendication 1, caractérisé en ce que les segments de principe actif sont agencés séparément dans l'espace des segments de matériau adhésif, qui sont constitués de systèmes de matériau adhésif tels, que ceux que l'on peut manier sous forme de dispersion, de plastisol ou d'organosol, et dans lequel aussi bien les segments de matériau adhésif que les segments de principe actif sont en forme approximativement de calotte et adhèrent par leur base au support.

10. Pansement auto-adhésif muni de segments de principe actif séparés sur un support enduit de matériau adhésif en vue de l'application transcutanée, selon la revendication 9, caractérisé en ce qu'il y a une feuille mince de séparation entre les segments de principe actif et la couche de matériau adhésif.

## Claims

1. Plaster, provided with a self-adhesive composition and containing an active compound, for transdermal administration of the active compound, characterized in that the active compound is moxonidine (2-methyl-4-chloro-6-methoxy-5-(2-imidazolin-2-yl)-amino-pyrimidine).

2. Plaster according to Claim 1, characterized in that it is a matrix plaster.

3. Plaster according to Claim 1, characterized in that the self-adhesive composition is selected from the group comprising the polyacrylate, polyisobutene, polyurethane, polysiloxane and polyvinyl alcohol compositions.

4. Plaster according to Claim 1, characterized by self-adhesive compositions based on acrylic and methacrylic acid esters with alkyl radicals having 4 to 18 carbon atoms.

5. Plaster according to Claim 1, characterized by self-adhesive compositions based on acrylic and methacrylic acid esters having a content of 0,1 to 10 per cent by weight of polar comonomers.

6. Plaster according to Claim 1, characterized by self-adhesive compositions based on polyisobutene/adhesive resin containing a high-molecular skeleton-forming fraction in addition to a low-molecular polyisobutylene fraction.

7. Plaster according to Claim 1, characterized by hydrophilic or polar substances as additives to the self-adhesive compositions.

8. Plaster according to Claim 1 for the treatment of hypertension.

9. Self-adhesive plaster having separate active compound segments on a carrier for transdermal administration, according to Claim 1, characterized in that, on the carrier and spatially separate from the active compound segments, adhesive segments are arranged which consist of those adhesive systems which can be processed as a dispersion, plastisol or organosol, both the adhesive segments and the active compound segments having an approximately hemispherical shape and adhering with their base to the carrier.

10. Self-adhesive plaster having separate active compound segments on an adhesive-coated carrier for transdermal administration, according to Claim 9, characterized in that there is a separating film between the active compound segments and the adhesive layer.